# EUROPEAN PATENT APPLICATION

(11) **EP 1 364 662 A1**
(43) Date of publication of application: **26.11.2003**
(21) Application number: 02010882.5
(22) Date of filing: 15.05.2002
(51) Int. Cl.: A61L 24/02, A61L 27/02

(54) **Gypsum composition for use in bone restoration operation with controlled working time**

(71) Applicant: CENTRAL MEDICAL TECHNOLOGIES INC., TAIPEI, TAIWAN R.O.C. (TW)
(72) Inventor: Chang, Han-Chao, Tainan (TW); Lin, Chih-I, Staten Island, NY 10304 (US); Say, Wen-Ching, Taipei (TW); Pan, Horng Wei, Keelung (TW); Lin, Shengfu, Taipei (TW)
(74) Representative: Grünecker, Kinkeldey, Stockmair & Schwanhäusser Anwaltssozietät

(57) **Abstract**

A gypsum composition is prepared to prolong the working time and shorten the setting time of the gypsum composition in a bone restoration operation. The gypsum composition is formed of a half-hydrate gypsum (CaSO₄·0.5H₂O), and an aqueous solution containing water, a polyol, and at least one solidification promoter selected from a polysaccharide, a polyethylene alcohol or mixtures thereof. The aqueous solution contains the water and the polyol in a volume ratio ranging between 25:1 and 2:1. The aqueous solution further contains 0.2% to 2.0% by weight of the solidification promoter.

## Description

### FIELD OF THE INVENTION

The present invention relates generally to a bone restoration operation, and more particularly to a method for use in the bone restoration operation to control the working time of a gypsum composition, and the nature of the gypsum composition.

### BACKGROUND OF THE INVENTION

The gypsum has been used as a bone substituent in the conventional surgical treatment of the bone deformities. The new surgical operation makes use of the gypsum as a bone substituent by injection. The technique disclosed in U.S. Pat. No. 6,251,139 is the case in point. The conventional gypsum composition is rather limited in its semi-solidification time and its working time. The phrase "the working time" refers to the duration between the time when the orthopedic gypsum is mixed with an aqueous solution to form a gypsum composition and the time at which the gypsum composition can hardly be ejected from a syringe. The phrase "the semi-solidification time" refers to the period of time starting from the time at which the orthopedic gypsum is mixed with an aqueous solution to the time at which the gypsum composition does not disperse in water or an aqueous solution such as physiological salt water, into which the gypsum composition is introduced. For example, the semi-solidification time of the conventional gypsum composition ranges from 2.5 minutes to 3.0 minutes, whereas the working time of the conventional gypsum composition ranges from 3.5 minutes to 4.0 minutes. In other words, the conventional gypsum composition will disperse in body liquid if it is injected into human body within 2.5 minutes starting from the time at which the orthopedic gypsum is mixed with an aqueous solution. It is likely that the conventional gypsum composition can not be injected into the body of a patient if the attending surgeon fails to administer the injection in 4.0 minutes starting from the time at which the orthopedic gypsum is mixed with an aqueous solution. Accordingly, only about one and a half minutes for the attending surgeon to complete the injection of the conventional gypsum composition into the body of a patient. It is conceivable that the attending surgeon is under pressure to rush the restorative operation at the expense of the well-being of the patient. It is therefore crucial to control the working time and the semi-solidification time of a gypsum composition.

### SUMMARY OF THE INVENTION

It is the primary objective of the present invention to provide a method for controlling the working time of an orthopedic gypsum composition.

It is another objective of the present invention to provide a method for controlling the setting time of an orthopedic gypsum composition.

It is still another objective of the present invention to provide an orthopedic gypsum composition which is free of the deficiencies of the conventional orthopedic gypsum composition.

The method of the present invention involves a first step in which an orthopedic gypsum is mixed with an aqueous solution to form a mixture, which is subsequently stirred evenly into a paste form. The weight ratio of the orthopedic gypsum and the aqueous solution ranges between 4:1 and 1:5. The method of the present invention is characterized by the aqueous solution which contains a polyol and at least one setting promoter selected from the group consisting of polysaccharide, poly(vinyl alcohol), and mixtures thereof. The volume ratio of water and polyol of the aqueous solution ranges between 25:1 and 2:1. The aqueous solution contains the setting promoter in the range of 0.2% to 2.0% by weight.

The agitation of the mixture of the orthopedic gypsum and the aqueous solution of the present invention may be attained by an agitation rod, magnetic agitator, agitation blade, or ultrasonic agitator.

The present invention also discloses gypsum composition comprising an orthopedic gypsum and an aqueous solution in a weight ratio of ranging from 4:1 to 1:5. The gypsum composition of the present invention is characterized by the aqueous solution which contains polyol and at least one solidification promoter selected from the group consisting of polysaccharide, poly(vinyl alcohol) and mixtures thereof. The volume ratio of water and polyol of the aqueous solution ranges between 25:1 and 2:1. The aqueous solution of the present invention contains 0.2-2.0% of the solidification promoter based on the weight of the aqueous solution .

If the composition of the present invention contains an excessive amount of the orthopedic gypsum, the composition is apt to fail to form a paste. If the composition of the present invention contains an insufficient amount of the orthopedic gypsum, the strength of the composition is reduced, and the composition may not be set in body liquid.

The aqueous solution may contain more than one polyols, but the volume ratio of the water and the polyols remains the same, i.e. between 25:1 and 2:1. If the aqueous solution contains an insufficient amount of water, the working time of the gypsum composition is likely shortened. If the aqueous solution contains an excessive amount of water, the setting time of the gypsum composition is prolonged to an extent that the injected gypsum composition is apt to deform or inadequate in strength. The volume ratio of the water and the polyol preferably ranges between 10:1 and 10:3. The optimal volume ratio of the water and the polyol of the aqueous solution of the present invention ranges between 20:3 and 4:1.

A suitable polyol for use in the present invention can be a water-soluble hydrocarbon compound having two or more than two hydroxyl groups, such as ethylene glycol, propylene glycol, glycerol, and polyethylene glycol. Glycerol is recommended.

The solidification promoter of the present invention may be a polysaccharide or a mixture of polysaccharides, poly(vinyl alcohol), a mixture of polysaccharide and poly(vinyl alcohol), with the polysaccharide or the mixture of polysaccharides being preferable. The amount of the promoter ranges between 0.2% and 2.0% based on the weight of the aqueous solution. If the amount of the promoter is excessively low, the gypsum composition so formed is apt to fail to form a paste upon being injected into the body of a patient. It is likely that the paste fails to form a set gypsum block of an appropriate strength in a period ranging between 0.5 and 1.0 hour. If the amount of the promoter is excessively large, the gypsum composition so formed will have a short working time. It is suggested that the amount of the promoter ranges between 0.4% and 1.5%, preferably 0.6% and 1.2% based on the total weight of the aqueous solution.

The polysaccharide may be starch or cellulose, preferably 1-4 linkage polysaccharides such as cellulose, methyl cellulose, ethyl cellulose, hydroxyethyl cellulose or mixtures thereof, preferably methyl cellulose, ethyl cellulose, or hydroxyethyl cellulose.

The gypsum composition of the present invention may contain a specific material which is intended to promote the growth of bone tissue, to relieve inflammation, or to enhance other effect, etc. If the specific material is solid, it can be mixed with the orthopedic gypsum, or can be dissolved in the aqueous solution. If the specific material is liquid, it is preferably dissolved in the aqueous solution in advance or along with the gypsum composition.

The orthopedic gypsum used in the present invention preferably is calcium sulfate half-hydrate (CaSO₄·0.5H₂O), abbreviated as half-hydrate gypsum.

### DETAILED DESCRIPTION OF THE PREFERRED EMBODIMENTS

The present invention will be more readily understood upon a thoughtful deliberation of the following comparative examples and the following non-restrictive embodiments.

### CONTROL EXAMPLES 1-10:

The half-hydrate gypsum (purchased from the Baker Co. of the U.S.) was mixed in a 20-milliliter syringe with the aqueous solution formed of water and glycerol, as shown in Table 1. The mixture was evenly stirred by a 316 stainless steel agitation rod having a length of 150 mm and a diameter of 1.0 mm. The working time and the setting time of the gypsum compositions so formed were measured and recorded in Table 1.

**TABLE 1**

| Control Example | Half-hydrate gypsum (g) | Water (ml) | Glycerol (ml) | Working time (min.) | Setting time (min.) |
|---|---|---|---|---|---|
| 1 | 10 | 0.5 | 0.5 | <2 | - |
| 2 | 10 | 1.0 | 0.5 | <2 | - |
| 3 | 10 | 1.5 | 0.5 | 3.0 | >60 |
| 4 | 10 | 2.0 | 0.5 | 9.5 | >60 |
| 5 | 10 | 2.5 | 0.5 | 12.0 | >60 |
| 6 | 10 | 3.0 | 0.5 | 12.5 | >60 |
| 7 | 10 | 3.5 | 0.5 | 11.5 | >60 |
| 8 | 10 | 4.0 | 0.5 | 17.5 | >60 |
| 9 | 10 | 4.5 | 0.5 | 25.0 | >60 |
| 10 | 10 | 5.0 | 0.5 | 26.5 | >60 |

### CONTROL EXAMPLES 11-20:

The procedures of Control Example 1 were repeated with various amounts of glycerol and 2.5 ml water in Table 2. The results are shown in Table 2.

**TABLE 2**

| Control Example | Half-hydrate Gypsum (g) | Water (ml) | Glycerol (ml) | Working time (min.) | Setting time (min.) |
|---|---|---|---|---|---|
| 11 | 10 | 2.5 | 0.1 | 4.5 | >60 |
| 12 | 10 | 2.5 | 0.2 | 6.0 | >60 |
| 13 | 10 | 2.5 | 0.3 | 7.0 | >60 |
| 14 | 10 | 2.5 | 0.4 | 9.5 | >60 |
| 15 | 10 | 2.5 | 0.5 | 11.5 | >60 |
| 16 | 10 | 2.5 | 0.6 | 10.5 | >60 |
| 17 | 10 | 2.5 | 0.7 | 16.5 | >60 |
| 18 | 10 | 2.5 | 0.8 | 20.5 | >60 |
| 19 | 10 | 2.5 | 0.9 | 26.5 | >60 |
| 20 | 10 | 2.5 | 1.0 | >30 | >60 |

### CONTROL EXAMPLES 21-30:

The procedures of Control Examples 11-20 were repeated except that propylene glycol was used in place of glycerol. The results are shown in Table 3.

**TABLE 3**

| Control Example | Propylene glycol (ml) | Working time (min.) | Setting time (min.) |
|---|---|---|---|
| 21 | 0.1 | 2.5 | >60 |
| 22 | 0.2 | 3.5 | >60 |
| 23 | 0.3 | 5.0 | >60 |
| 24 | 0.4 | 7.0 | >60 |
| 25 | 0.5 | 9.0 | >60 |
| 26 | 0.6 | 12.5 | >60 |
| 27 | 0.7 | 15.0 | >60 |
| 28 | 0.8 | 17.5 | >60 |
| 29 | 0.9 | 25.0 | >60 |
| 30 | 1.0 | >30 | >60 |

### CONTROL EXAMPLES 31-40:

In Control Examples 31-40, polyethylene glycol #400 was used in place of glycerol in the control examples 11-20. The polyethylene glycol #400 has an average molecular weight of about 400. The results are shown in the following Table 4.

**TABLE 4**

| Control Example | Polyethylene glycol (ml) | Working time (min.) | Setting time (min.) |
|---|---|---|---|
| 31 | 0.1 | 3.5 | >60 |
| 32 | 0.2 | 5.0 | >60 |
| 33 | 0.3 | 5.0 | >60 |
| 34 | 0.4 | 5.5 | >60 |
| 35 | 0.5 | 6.0 | >60 |
| 36 | 0.6 | 7.5 | >60 |
| 37 | 0.7 | 6.5 | >60 |
| 38 | 0.8 | 8.5 | >60 |
| 39 | 0.9 | 9.5 | >60 |
| 40 | 1.0 | >10 | >60 |

### EXAMPLES 1-5

A mixture of gypsum composition was formed in a 20-ml syringe by 10 grams of half-hydrate gypsum and an aqueous mixed solution containing glycerol (as the polyol) and a methyl cellulose solution (as the solidification promoter). The mixture was thoroughly stirred by means of an agitation rod made of 316 stainless steel. Thereafter, the working time and the setting time of the gypsum composition were measured. The amounts of water, glycerol and the methyl cellulose solution used together with the results are shown in the following Table 5.

It must be noted here that the methyl cellulose solution refereed to above was prepared by adding 4 grams of methyl cellulose to 100 milliliters of water, heating the resulting mixture to reach the boiling state and was then cooled.

**TABLE 5**

| Example | Half-hydrate gypsum (g) | Water (ml) | Glycerol (ml) | Methyl cellulose solution (ml) | Working time (min.) | Setting time (min.) |
|---|---|---|---|---|---|---|
| 1 | 10 | 2.0 | 0.1 | 0.5 | 4.0 | 4.5 |
| 2 | 10 | 2.0 | 0.3 | 0.5 | 7.0 | 10.5 |
| 3 | 10 | 2.0 | 0.5 | 0.5 | 9.0 | 13.0 |
| 4 | 10 | 2.0 | 0.7 | 0.5 | 14.0 | 19.5 |
| 5 | 10 | 2.0 | 0.9 | 0.5 | 17.5 | 28.0 |

### EXAMPLES 6-12:

The Examples 6-12 are basically similar in preparation to the Example 3, with the difference being that the polyol (0.5 ml) and the solidification promoter (0.5 ml) of the Examples 6-12 were varied, as shown in the following Table 6.

**TABLE 6**

| Ex. | Polyol (ml) | Cellulose solution (ml) | Working time (min.) | Setting time (min.) |
|---|---|---|---|---|
| 6 | glycerol | ethyl cellulose | 7.0 | 13.5 |
| 7 | glycerol | hydroxyethyl cellulose | 8.0 | 15.0 |
| 8 | glycerol | methyl cellulose + hydroxyethyl cellulsoe (1:1) | 7.5 | 15.5 |
| 9 | propylene glycol | hydroxyethyl cellulose | 6.5 | 14.0 |
| 10 | propylene glycol | ethyl cellulose + hydroxyethyl cellulose (1:1) | 7.5 | 18.5 |
| 11 | polyethylene glycol #400 | hydroxyethyl cellulose | 3.5 | 6.5 |
| 12 | polyethylene glycol #400 | methyl cellulose + hydroxyethyl cellulsoe (1:1) | 3.5 | 6.5 |

### EXAMPLES 13-15:

Examples 13-15 are basically similar in preparation to Example 3, except that the solidification promoters (0.5 ml) of Examples 13-15 were changed to a poly(vinyl alcohol) solution, a sticky rice starch solution and a corn starch solution, respectively. The poly(vinyl alcohol) solution was a glue available in the market under trademark SIMBALION®, from Lion Pencil Co., Taiwan. The sticky rice starch solution was prepared by dissolving 3 grams of sticky rice starch or corn starch available from market in 100 milliliters of water. The mixture of the starch and the water was heated to reach the boiling state and was then cooled to become a viscous liquid.

**TABLE 7**

| Example | Working time (min.) | Setting time (min.) |
|---|---|---|
| 13 | 6.0 | 10.0 |
| 14 | 5.0 | 8.5 |
| 15 | 6.5 | 10.0 |

## Claims

1. A gypsum composition for use in a bone restoration operation comprising an orthopedic gypsum and an aqueous solution in a weight ratio of said orthopedic gypsum to said aqueous solution ranging from 4:1 to 1:5, wherein said aqueous solution comprises water, a polyol, and at least one solidification promoter selected from the group consisting of a polysaccharide, a poly(vinyl alcohol) or a mixture of them, wherein a volume ratio of said water to said polyol in said aqueous solution ranges from 25:1 to 2:1, and said aqueous solution contains 0.2% to 2.0% of said solidification promoter based on the weight of said aqueous solution.

2. The gypsum composition as defined in claim 1, wherein said volume ratio of said water to said polyol ranges substantially from 10:1 to 10:3.

3. The gypsum composition as defined in claim 2, wherein said volume ratio ranges substantially from 20:3 to 4:1.

4. The gypsum composition as defined in claim 1, wherein said polyol is glycerol, ethylene glycol, propylene glycol, or polyethylene glycol.

5. The gypsum composition as defined in claim 4, wherein said polyol is glycerol.

6. The gypsum composition as defined in claim 1, wherein said aqueous solution contains 0.4% to 1.5% of said solidification promoter based on the weight of said aqueous solution.

7. The gypsum composition as defined in claim 6, wherein said aqueous solution contains 0.6% to 1.2% of said solidification promoter based on the weight of said aqueous solution.

8. The gypsum composition as defined in claims 1, 4 or 5, wherein said solidification promoter is methyl cellulose, ethyl cellulose, or hydroxyethyl cellulose.

9. The gypsum composition as defined in any one of claims 1-8, wherein said orthopedic gypsum is calcium sulfate half-hydrate (CaSO₄·0.5H₂O).
